Europäisches Patentamt

European Patent Office

Office européen des brevets

(11) Publication number: **0 452 912 A2**

# EUROPEAN PATENT APPLICATION

(21) Application number: **91106163.8**

(22) Date of filing: **17.04.91**

(51) Int. Cl.5: **A61M 5/155**

(30) Priority: **19.04.90 JP 103510/90**

(43) Date of publication of application:
**23.10.91 Bulletin 91/43**

(84) Designated Contracting States:
**DE FR GB IT**

(71) Applicant: **NISSHO CORPORATION**
**9-3, Honjo-nishi 3-chome Kita-ku**
**Osaka-shi(JP)**

(72) Inventor: **Tsujikawa, Hajime**
**5-19, Hama-cho**
**Otsu-shi, Shiga-ken(JP)**
Inventor: **Hiejima, Katsuhiro**
**1621, Kasayama, Minakasa-machi**
**Kusatsu-shi, Shiga-ken(JP)**

(74) Representative: **Türk, Gille, Hrabal**
**Brucknerstrasse 20**
**W-4000 Düsseldorf 13(DE)**

(54) **Liquid infusion device.**

(57) A liquid infusion device comprising a bladder assembly including a bladder, a housing containing the bladder assembly, an inlet/outlet portion provided on the one end of the housing, a liquid drug passage, and a flow-regulating portion provided at the liquid drug passage. The bladder comprises a tubular body made of natural rubber and a layer made of silicone resin covering an inner surface of the tubular body. In the liquid infusion device, internal pressure of a bladder when dispensing liquid durg is high, so that liquid drug can be dispensed to a patient at about a constant velocity. Additives in natural rubber do not exude since liquid drug in the bladder does not contact with natural rubber.

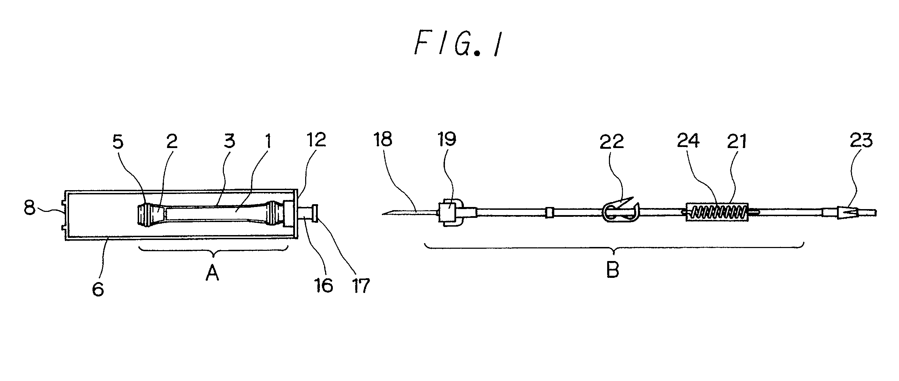

$FIG. 1$

The present invention relates to a liquid infusion device used for continuously dispensing a predetermined amount of liquid drug little by little to blood vessel, urinary bladder and the like, and more particularly to a liquid infusion device capable of continuously dispensing liquid drug contained in a bladder in a pressured condition to a patient little by little at a constant velocity for a long period of time.

Hitherto, as a device for dispensing a very small amount of liquid drug such as antibiotics and carcinostatic substance to blood vessel, urinary bladder and the like, there is proposed a liquid infusion device (Japanese Unexamined Patent Publication No. 108790/1975 corresponding to USP 3,895,631) wherein liquid drug is charged into a bladder made of elastic material and liquid drug is dispensed continuously into blood vessel and the like for a long period of time by shrinkage force of the bladder. Further, one of the present inventors proposed a liquid infusion device (Japanese Unexamined Patent Publication No. 135360/1989 corresponding to USP 4,909,790) wherein silicone resin is used as a material for a bladder and liquid drug charged into the bladder is dispensed at about a constant velocity into a patient.

The above liquid infusion device using a bladder made of silicone resin is, however, liable to be influenced by external surroundings because of low internal pressure of the bladder. Accordingly, flow rate of liquid drug changes so that liquid drug cannot be dispensed at a constant velocity when dispensing liquid drug to artery or vein. Further, initial dispensation velocity of liquid drug is larger than the average dispensation velocity. In the case of some kind of liquid drug, large dispensation amount of liquid drug in a short period of time is in danger of exerting a bad influence on a patient.

An object of the present invention is to provide a liquid infusion device capable of dispensing liquid drug to a patient at a constant velocity.

In accordance with the present invention, there is provided a liquid infusion device comprising

(a) a bladder assembly including a bladder made of elastic material, the bladder containing liquid drug in a pressured condition, and having an opening into which liquid drug is charged and from which liquid drug is dispensed,

(b) a housing containing the bladder assembly, and having an opening at one end of the housing,

(c) an inlet/outlet portion provided on the one end of the housing and communicating to the opening of the housing,

(d) a liquid drug passage extending from the inlet/outlet portion, and

(e) a flow-regulating portion provided at the liquid drug passage for regulating flow rate of liquid drug

wherein the bladder has a multilayer structure of a tubular body made of natural rubber and a silicone resin layer covering an inner surface of the tubular body.

The present invention further provides a liquid infusion device wherein ratio of a thickness of the tubular body made of natural rubber to a thickness of the layer made of silicone resin is from 1.5 to 10 in the bladder having a multilayer structure.

In a liquid infusion device of the present invention which dispenses liquid drug charged within a bladder to a patient by shrinkage force of the bladder, the bladder has a multilayer structure. That is, the bladder comprises a tubular body made of natural rubber having a large stress when being extended as a base layer, and a layer made of chemical resistant silicone resin covering the inner surface of the tubular body. The above-mentioned multilayer structure acts to prevent exudation of additives in natural rubber into liquid drug, and liquid drug in the bladder can be dispened to a patient at about a constant velocity for a long period of time since natural rubber has small inner pressure hysteresis and small stress relaxation.

Fig. 1 is an explanatory view of an embodiment of a liquid infusion device of the present invention;

Fig. 2 is an enlarged sectional view of a bladder assembly of the liquid infusion device of Fig. 1 wherein liquid drug is charged into a bladder;

Figs. 3A and 3B are explanatory sectional views of bladders having a multilayer structure;

Fig. 4 is a graph showing stress-strain curve of a bladder in the present invention; and

Fig. 5 is a graph showing change of dispensation velocity of bladders in Fig. 4.

Referring now to the accompanying drawing, there is explained a liquid infusion device of the present invention. In Figs. 1, 2, 3A, and 3B, symbol A is a bladder assembly, symbol B is a liquid drug dispensation assembly, numeral 1 is an outer shaft, numeral 2 is an inner shaft, numeral 3 is a bladder, numeral 6 is a housing, numeral 10 is a plug, numeral 18 is an injection needle, numeral 21 is a flow-regulating portion, numeral 22 is a cramp, numeral 31 is a layer of natural rubber, and numeral 32 is a layer of silicone resin.

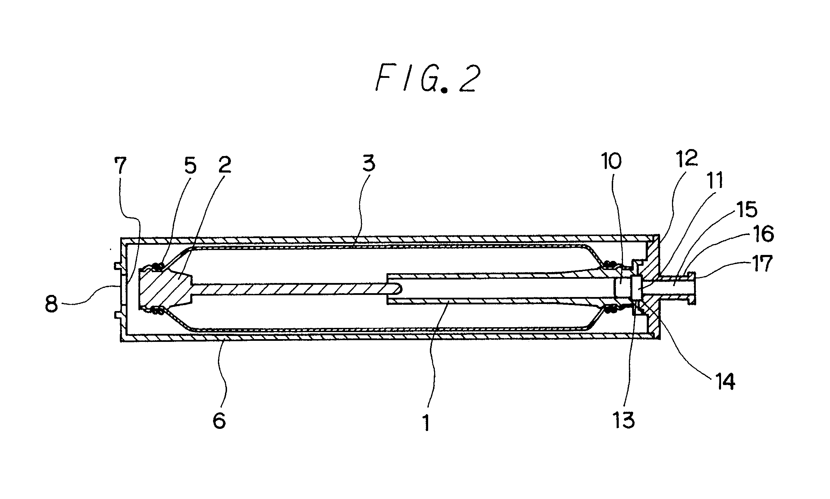

In Figs. 1 and 2, the bladder assembly A is a portion storing or containing liquid drug therein and injecting liquid drug to a predetermined portion of human body, and comprises a tubular outer shaft 1, an inner shaft slidably received within the outer shaft 1, a bladder 3 placed outside the outer shaft 1 and inner shaft 2, and a plug 10 connected to one end of the outer shaft opposite to an end wherefrom the inner shaft 2 is inserted. The outer shaft 1 and inner shaft 2 are made of synthetic resin such as polycarbonate, polyethylene, and polypropylene.

The bladder 3 having a tubular shape is placed outside the outer shaft 1 and inner shaft 2 to cover both shafts. One end of the bladder 3 is airtightly fixed to the outer shaft 3 by sealing means such as O-ring 5 or metalic spiral stopper while the other end of the bladder 3 is similarly fixed to the inner shaft 2. The inside of the outer shaft 1 wherein the inner shaft 2 slides serves as a passage for liquid drug charged within the bladder 3 to a human body. It is accordingly preferable to provide clearance of about 0.5 to 3 mm between an inner surface of the outer shaft 1 and an outer surface of the inner shaft 2. The size and thickness of the bladder 3 are not particularly limited in the liquid infusion device of the present invention, therefore, bladders having various kinds of size and thickness can be applied to a liquid infusion device of the present invention depending on the amount of liquid drug dispensed to patients, dispensation time and the like. The employable values of outer diameter, thickness, and length thereof are about 2 to 30 mm, 0.1 to 2.0 mm, and 3 to 30 cm, respectively. The bladder 3 is inflatable in both radial direction and longitudinal direction (i. e. axial direction of the outer shaft 1 and inner shaft 2) by charging of liquid drug. The inner shaft 2 goes into or goes out from the outer shaft 1 with the movement of the bladder 3. An amount of liquid drug dispensed from the bladder 3 can be determined by marking with degrees at the surface of a housing stated below, since the relationship between position of the inner shaft 2 and an amount of liquid drug remained in the bladder 3 is constant.

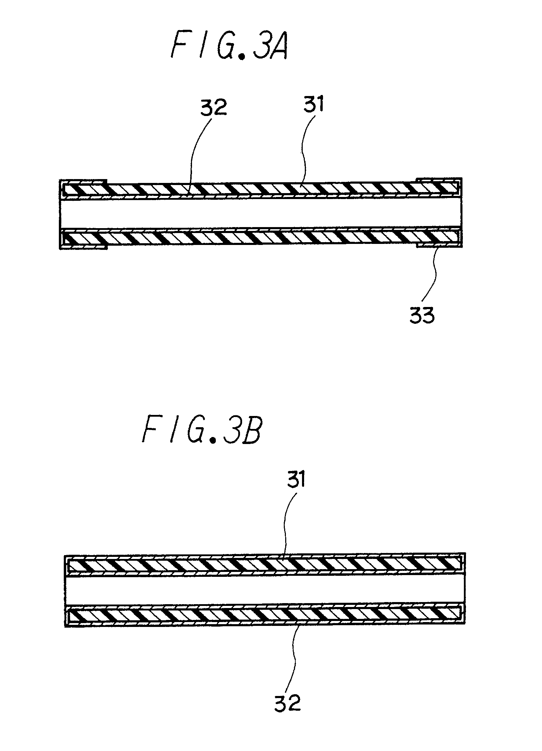

The bladder 3 has a multilayer structure, as shown in Fig. 3A or Fig. 3B, in which the inner surface of a tubular body 31 made of natural rubber is covered with a layer 32 made of silicone resin.

The tubular body 31 made of natural rubber is obtained by adding sulfur, zinc oxide, vulcanization accelerator and the like to natural rubber, and forming the resulting material into a tube by extrusion molding, injection molding and the like. In that case, a part of additives in natural rubber might be removed with the use of solvents, or antioxidants might be added to natural rubber after removal of additives.

Concrete examples of silicone resin are, for example, resins having organosiloxane combination such as dimethylpolysiloxane, dimethylvinylpolysiloxane, methylphenylvinylpolysiloxane, and methylphenylpolysiloxane. Additives such as vulcanizing agents, dispersion accelerators, and reinforcing fillers are added to these resins.

A bladder 3 shown in Fig. 3A is produced by inserting a tube made of silicone resin into a hollow portion of a tube made of natural rubber, and folding both ends of the tube made of silicone resin at both ends of the tube made of natural rubber to form folded-back portions 33. Both ends of the obtained multilayer tube are fixed to the outer shaft 1 and inner shaft 2 as stated above.

A bladder in the present invention can be produced by other methods. For example, a bladder shown in Fig. 3B is produced by dipping a tube made of natural rubber into a solution prepared by disolving silicone resin in organic solvent and coating the inner surface, outer surface and both end surfaces of the tube made of natural rubber with silicone resin layer 32.

Ratio of the thickness of the natural rubber layer 31 to that of the silicone resin layer 32 is preferably from 1.5 to 10, more preferably from 3 to 6. When the ratio is less than 1.5, stress of the bladder when being extended becomes small so that flow rate of liquid drug tends to change and accordingly dispensation of liquid drug at a constant velocity becomes difficult. On the other hand, when the ratio is more than 10, the thickness of a bladder becomes large, so taht the charging of liquid drug into the bladder tends to becomes difficult by large internal pressure of bladder.

The thickness of the silicone resin layer is preferable from 100 to 500 $\mu$m, more preferably from 200 to 400 $\mu$m. When the thickness of the silicone resin layer is more than 500 $\mu$m, the thickness of a bladder becomes large, so that the charging of liquid drug into the bladder tends to become difficult. On the other hand, when the thickness is less than 100 $\mu$m, there is a danger that additives contained in the natural rubber layer 31 exudes into liquid drug by expansion of the bladder caused by injection of liquid drug.

The housing 6 serves not only to prevent damage of the bladder 3 by the contact with external sharp objects but also to seal liquid drug so as not to disperse out when it leaks out from the bladder because of defects of the bladder such as pinhole. The housing 6 is made of synthetic resin such as polyvinyl chloride, polypropylene and polycarbonate. It is preferable that the housing 6 is made of transparent material to allow observation of liquid drug dispensed with naked eyes from outside the housing 6.

As described above, the housing 6 covers the bladder assembly A in a sealed condition to prevent leak-out of liquid drug even if the bladder 3 is damaged. There is arised a disadvantage, however, if the inside of the housing 6 is perfectly kept airtight, that air pressure in the housing 6 becomes high with the injection of liquid drug into the bladder 3 so that the injection of liquid drug beyond some volume becomes impossible. To avoid the above disadvantage, there is formed an opening 7 for air vent at a suitable place of the housing 6 in the liquid infusion device of the present invention. A hydrophobic filter 8, which allows passage of air but not liquid drug, is provided at the opening 7. As a material for the hydrophobic filter 8, polyester, fluororesin, or a laminate of polyester and fluororesin can be used.

A plug 10 in airtightly and liquidtightly inserted into an end of the outer shaft 1 (such an end opposite to the end whereinto the inner shaft 2 is inserted) and is used for injecting liquid drug into the bladder. The plug 10 is made of rubber-like elastic material such as silicone rubber and has a superior prick resistance. In the specification the term "prick resistance" means a property which keeps liquidtightness even if pricked with an injection needle for many times, and which prevents the leak of liquid drug in the bladder. The plug 10 shown in Fig. 2 has a head 11. Stepped portions 13, 14 are formed on the end of the outer shaft 1 and the end plate 12 of a housing 6 respectively. The head 11 of the plug 10 is fixed by the above-mentioned stepped portion 13, 14, so that slippage of coming out of the plug 10 is prevented when pricking the plug with a needle or pulling the needle out to inject or dispense liquid drug. In Fig. 1, the plug 10 functions as both an inlet and an outlet of liquid drug.

A hole 15 used for inserting an injection needle thereinto is formed on the end plate 12 of the housing 6. On the end plate 12 of the housing 6, there is formed an approximately tubular projection 16 coaxial with the hole 15. The projection 16 has a flange 17 to be connected to a connector 19 provided on the injection needle side of the liquid drug dispensation assembly B. The connection between the housing 6 and the liquid drug dispensation assembly B can be carried out by pricking the plug 10 with the injection needle 18. Secure connection between the bladder assembly A and liquid drug dispensation assembly B, however, can be performed by additionally engaging the projection 16 with or screwing the projection 16 into the connector 19. When injecting liquid drug into a bladder, a plug 10 is pricked with an injection needle with the use of an injector and the like (not shown) and liquid drug in the injector is injected into the bladder 3.

In addition to the above-mentioned injection needle 18, the liquid drug dispensatiton assembly B has a flow-regulating portion 21, a cramp 22 capable of stopping flow of liquid drug on occasion, and a connector 23.

The flow-regulating portion 21 serves to regulate flow rate of liquid drug, and comprises a very thin pipe. As a material for the pipe, metal such as stainless steel, ceramic, or synthetic resin such as polyolefine, polyvinylchloride and polyester can be used. The inner diameter and length of the pipe a preferably from 10 to 500 $\mu$m and from 1 to 5000 mm respectively. When the inner diameter of the pipe is less than 10 $\mu$m, flow of liquid drug tends to stop due to entrance of air in liquid drug. On the other hand, when the inner diameter of the pipe is more than 500 $\mu$m, control of flow rate of liquid drug tends to become difficult. When the length of the pipe is less than 1 mm, control of flow rate of liquid drug tends to become difficult. On the other hand, when the length of the pipe is more than 5000 mm, size of a device becomes too large. In that case, however, the flow-regulating portion 21 can be miniaturized by arranging a pipe 24, which has a spring-like or spiral structure capable of extending and shrinking in an axial direction to some extent, in a casing.

A tapered connector 23 is attached to the end of the liquid drug dispensation assembly B. Through the connector 23, a vein needle or a PSV assembly is connected to the liquid drug dispensation assembly B. A check value (not shown) might be mounted in the connector 23 to prevent a back flow of liquid drug due to vein pressure.

Next, there is explained a use of a liquid infusion device of the present invention based on Fig. 1.

The injection of liquid drug is carried out by pricking the plug 10 with, for example, an injection needle of an injector and charging liquid drug into the bladder 3. After a predetermined amount of liquid drug is charged into the bladder 3, the injector is pulled out from the plug 10. Then the plug 10 is pricked with the needle 18 of the liquid drug dispensation assembly B to connect the bladder assembly A to the liquid drug dispensation assembly B. In that case, the cramp 21 is required to be closed so as to prevent flow of liquid drug toward a patient. Thereafter, liquid drug in the bladder 3 is dispensed into the body of a patient by stress of the bladder while regulating flow rate of liquid drug by the flow-regulating portion 21.

Example 1

A tube (inner diameter: 5.0 mm, outer diameter: 7.0 mm, length: 43 mm) was produced by extruding natural rubber produced by Komine Rubber Industry Co., Ltd. The obtained tube was heated in a mixed solvent of acetone and hexane (mixing ratio: 1 to 2, temperature: 60°C ) for two hours to extract additives in natural rubber such as sulfur and vulcanization accelerator. Then, the tube made of natural rubber was dipped into ethanol solution of hideredphenol (antioxidant) (concentration: 0.02 g/m$\ell$ ) for four hours, and dried. Into a hollow portion of the treated tube made of natural rubber, a tube made of silicone resin available from Shin-Etsu Chemical Co., Ltd. under the commercial name of KE 77 (inner diameter: 4.6 mm, outer diameter: 5.0 mm, length: 63 mm) was inserted. Both ends of the tube made of silicone resin were folded back at both ends of natural rubber tube toward the outer surface of natural rubber tube as shown in Fig. 3A. A bladder is assembled by fixing both ends of the obtained multilayer tube to ends of the inner shaft 1 and outer shaft 2 respectively by O-rings. The bladder assembly was assembled into a liquid infusion device shown in Fig. 1 together with other parts. 60 m $\ell$ of water was charged into the bladder through the plug 10 by using an injector.

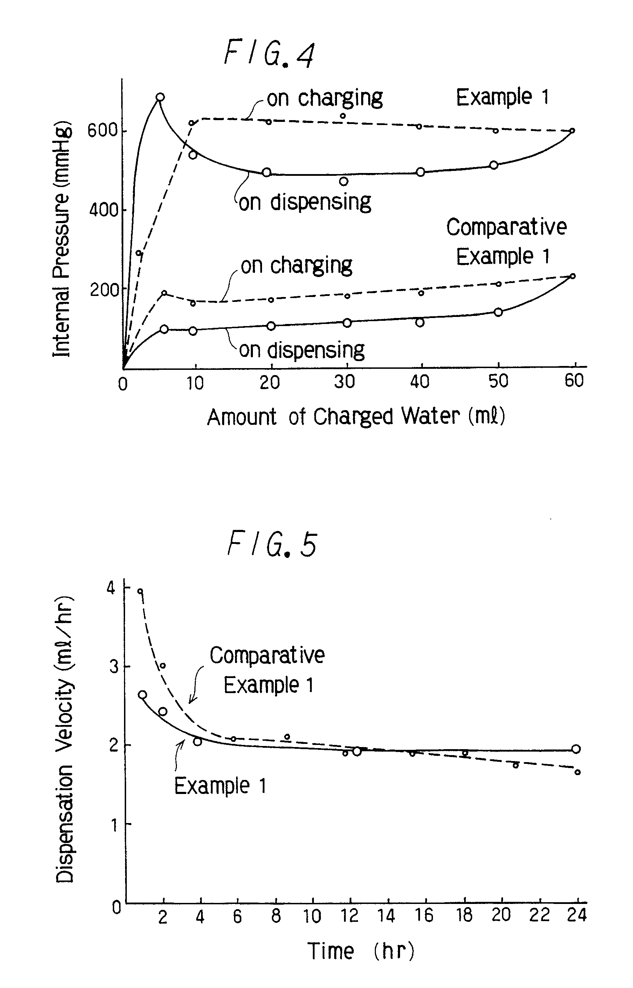

Next, water in the bladder was dripped from a vein needle attached to a connector 19 using a flow-regulating portion in which a very thin spiral stainless pipe (outer diameter: 0.3 mm, inner diameter: 0.1 mm, total length: 250 mm) was encased in a casing. Fig. 4 is a graph showing stress-strain curve of a bladder. The graph shows the relationship between an amount of liquid drug and an internal pressure of the bladder on charging and dispensing liquid drug. The internal pressure of the bladder was measured by gange pressure of a pressure monitor produced by Cosmo Keiki Co., Ltd. under the commercial name of DP-310. Fig. 5 shows the relationship between dispensation time (the time elapsed) and dispensation velocity.

Comprative Example 1

The procedure of Example 1 was repeated except that a tube made of the same silicone resin as in Example 1 (inner diameter: 5.0 mm, outer diameter: 7.0 mm, length: 63 mm) was used as a bladder instead of the multilayer tube in Example 1. The relationship between an amount of liquid drug and an internal pressure of the bladder on charging and dispensing liquid drug is shown in Fig. 4. Further, the relationship between dispensation time (the time elapsed) and dispensation velocity is shown in Fig. 5.

As is clear from Fig. 4 and 5, the liquid infusion device of Example 1 having a multilayer structure has higher internal pressure on dispensing liquid drug and smaller initial dispensation velocity than the liquid infusion device of Comparative Example 1. Accordingly, the liquid infusion device of Example 1 can dispense liquid drug at about a constant velocity for a long period of time.

Examples 1 to 3 and Comparative Example 2

Three bladders were produced wherein the thickness of the tube made of silicone resin in Example 1 was changed as shown in Table 1. Each bladder was incorporated into the liquid infusion device of Example 1. After 60 m $\ell$ of water was charged into the bladder, water in the bladder was dispensed for 24 hours. Ratio of initial dispensation velocity (dispensation velocity in first two hours, i. e. from the beginning of dispensation to two horus after the beginning of dispensation) to average dispensation velocity is shown in Table 1.

Table 1

| No. | Thickness of silicone resin tube S (mm) | Thickness of natural rubber tube N (mm) | Ratio of thickness N/S | Average dispensation velocity M (ml/hr) | Initial dispensation velocity F (ml/hr) | Ratio of velocity F/M |
|---|---|---|---|---|---|---|
| Ex. 1 | 0.2 | 1.0 | 5 | 2.03 | 2.55 | 1.26 |
| Ex. 2 | 0.1 | 1.0 | 10 | 2.04 | 2.44 | 1.20 |
| Ex. 3 | 0.5 | 1.0 | 2 | 2.06 | 3.05 | 1.48 |
| Com. Ex. 2 | 0.8 | 1.0 | 1.25 | 2.07 | 3.36 | 1.62 |

As is clear from Table 1, ratio of velocity becomes small with increase of ratio of tube thickness, so that liquid drug can be dispensed at about a constant dispensation velocity. However, injection of liquid drug into the bladder tends to become difficult.

On the other hand, with decrease of ratio of tube thickness, initial dispensation velocity tends to become large and dispensation velocity of the bladder tends become unstable.

As stated above, according to a liquid infusion device of the present invention, a bladder has a multilayer structure in which the inner surface of a tubular body made of natural rubber is covered with a layer made of silicone resin. Accordingly, internal pressure of a bladder when dispensing liquid drug is high, so that liquid drug can be dispensed to a patient at about a constant velocity. Further, in the liquid infusion device of the present invention, additives in natural rubber do not exude since liquid drug in the bladder does not contact with natural rubber.

## Claims

1. A liquid infusion device comprising
   (a) a bladder assembly including a bladder made of elastic material, the bladder containing liquid drug in a pressured condition, and having an opening into which liquid drug is charged and from which liquid drug is dispensed,
   (b) a housing containing the bladder assembly, and having an opening at one end of the housing,
   (c) an inlet/outlet portion provided on the one end of the housing and communicate to the opening of the housing,
   (d) a liquid drug passage extending from the inlet/outlet portion, and
   (e) a flow-regulating portion provided at the liquid drug passage for regulating flow rate of liquid drug
   wherein the bladder has a multilayer structure of tubular body made of natural rubber and a silicone resin layer covering an inner surface of the tubular body.

2. The device of Claim 1, wherein ratio of a thickness of the tubular body made of natural rubber to a thickness of the layer made of silicone resin is from 1.5 to 10.

# F I G. I

F I G. 2

# FIG.3A

32    31

33

# FIG.3B

31

32

## F I G. 4

## F I G. 5